# EUROPEAN PATENT APPLICATION

(11) **EP 4 625 430 A1**
(43) Date of publication of application: **01.10.2025**
(21) Application number: 23921455.4
(22) Date of filing: 31.08.2023
(51) Int. Cl.: G16H 20/70, G16H 50/20, G16H 10/20, H04L 51/02, G16H 10/60

(54) **APPARATUS FOR EVALUATING ETHICALITY OF ARTIFICIAL INTELLIGENCE MODEL ACCORDING TO USER TYPE**

(30) Priority: 06.02.2023 KR 20230015556; 30.08.2023 KR 20230114453
(71) Applicant: Moon, Manki, Yangju-si Gyeonggi-do 11476 (KR)
(72) Inventor: Moon, Manki, Yangju-si Gyeonggi-do 11476 (KR)
(74) Representative: BCKIP Part mbB
(86) International application number: PCT/KR2023/013006
(87) International publication number: WO 2024/167081

(57) **Abstract**

An electronic device according to an embodiment of the present invention may be configured by including: a memory for storing an evaluation criterion related to ethicality of an artificial intelligence model performing a consultation; and a processor that measures an ethicality degree of result data output by the artificial intelligence model according to the evaluation criterion, wherein the ethicality degree is measured according to types of users performing consultations with the artificial intelligence model.

## Description

### Technical Field

The present disclosure relates to an apparatus for evaluating ethicality of an artificial intelligence model according to user types.

### Background Art

With the rapid development of psychological consultation technology based on artificial intelligence, AI-based psychological consultation robots or apps are expected to greatly benefit the general public.

The AI-based psychological consultation technology has several advantages in terms of consultation fees and accessibility compared to traditional human counselor-based consultation methods. Accordingly, the AI-based psychological consultation technology may allow low-income users or users who are reluctant to disclose sensitive personal issues to more freely access psychological consultation.

However, since such AI-based psychological consultation technologies are implemented in the form of a black box, issues related to the ethicality of the output data may arise. Therefore, there is a need for further research on methods for implementing artificial intelligence models that provide user-friendly responses and explanations and evaluating the ethicality of the artificial intelligence models.

### Disclosure

### Technical Problem

An embodiment of the present disclosure has been made to provide a consultation model customized to user characteristics based on explainable artificial intelligence (XAI).

An embodiment of the present disclosure has been made to classify consultation subjects into one of multiple types based on XAI and to evaluate the ethicality of an artificial intelligencemodel performing consultation according to user types.

The objectives to be achieved by the present disclosure are not limited to the above-mentioned objectives, and other objectives which are not mentioned will be more clearly understood by those skilled in the art from the following description and the embodiments of the present disclosure. Furthermore, it will be readily apparent that the objectives and advantages of the present disclosure can be achieved by the means and combinations thereof set forth in the claims.

### Technical Solution

To accomplish the above-mentioned objectives, according to the present disclosure, there is provided an electronic device including:a memory that stores evaluation criteria related to the ethicality of an artificial intelligence model performing consultation; anda processor that measures an ethicality degree of result data output by the artificial intelligence model according to the evaluation criteria, wherein the ethicality degree is measured according to user types of users consulting with the artificial intelligence model.

Moreover, the processor may include a user determination unit that measures scores for respective ethicality-related items of the user based on the consultation content between the artificial intelligence model and the user, and classifies the user byuser types based on the item scores.

In this instance, the user types may be classified based on scores for items including disposition, virtue, personality, cognitive faculty, and personal environments. The disposition refers to a property regarding consistency, the virtue refers to a property regarding morality, the personality refers to a property regarding empathy, the cognitive faculty refers to a property regarding problem-solving ability, and the personal environments refer to a property related to social support.

Furthermore, the processor may include an ethicality determination unit measuring scores for respective ethicality-related items of the artificial intelligence model based on the consultation content between the artificial intelligence model and the user. The ethicality-related items of the artificial intelligence model may include interpretability, transparency, responsibility, bias, and stability.

The ethicality determination unit may measure the ethicality score of the artificial intelligence model based on a user's survey regarding results output by the artificial intelligence model or measure the ethicality score of the artificial intelligence modelbased on analysis of the user's condition changes after the consultation with the artificial intelligence model.

In another aspect of the present disclosure, a control method of an electronic device according to various embodiments of the present disclosure may include: performing consultation between an artificial intelligence model and a user; identifying the user type based on the consultation content; andmeasuring an ethicality degree of result data output by the artificial intelligence model according to pre-stored evaluation criteria, wherein the ethicality degree is measured according to the identified user type.

In another aspect of the present disclosure, a computer program stored in a computer-readable recording medium according to an embodiment of the present disclosure may be executed by a processor of an electronic device to perform the above control method.

### Advantageous Effect

According to an embodiment of the present disclosure, the apparatus for evaluating ethicality of an artificial intelligence model according to user types can evaluate the ethicality of the artificial intelligence model performing a consultation corresponding to user characteristics, thereby providing a user-customizedconsultation model.

Additionally, according to an embodiment of the present disclosure, the apparatus for evaluating ethicality of an artificial intelligence model according to user types can collect user personality datathrough a Q&A process with the explainable artificial intelligence (XAI)-based consultation model, thus performing user type identificationin an environment similar to real consultations and reducing user fatigue without requiring a separate test.

In addition, according to an embodiment of the present disclosure, the apparatus for evaluating ethicality of an artificial intelligence model according to user types can perform consultation based on the explainable artificial intelligence (XAI) to provide human-friendly explanations of diagnostic results acceptable to consultation subjects and guidelines, thus improving user satisfaction.

### Description of Drawings

FIG. 1 is a diagram illustrating the configuration of an electronic device according to an embodiment of the present disclosure.

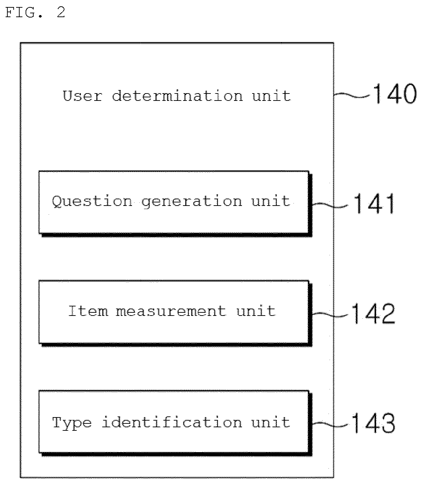

FIG. 2 is a diagram illustrating the configuration of a user determination unit according to an embodiment of the present disclosure.

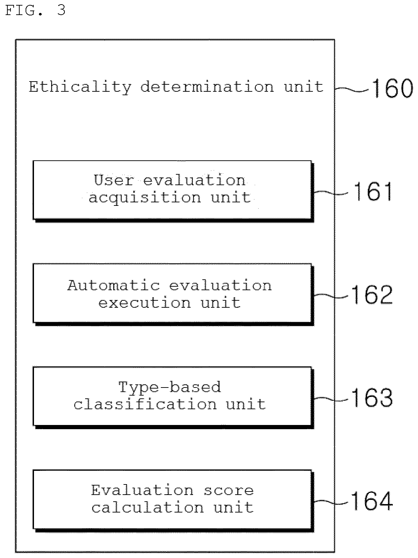

FIG. 3 is a diagram illustrating the configuration of an ethicality determination unitaccording to an embodiment of the present disclosure.
FIG. 4 is a diagram for depicting an evaluation operation for output data of an artificial intelligence model according to user types according to an embodiment of the present disclosure.
FIGS. 5 and 6 are diagrams illustrating the evaluation operation of the artificial intelligence model of the electronic device according to an embodiment of the present disclosure.

### Best Mode

According to an embodiment of the present disclosure, an electronic device may include a memory that stores evaluation criteria related to the ethicality of an artificial intelligence model performing consultation; anda processor that measures an ethicality degree of result data output by the artificial intelligence model according to the evaluation criteria, wherein the ethicality degree is measured according to user types of users consulting with the artificial intelligence model.

Moreover, the processor may include a user determination unit that measures scores for respective ethicality-related items of the user based on the consultation content between the artificial intelligence model and the user, and classifies the user byuser types based on the item scores.

In this instance, the user types may be classified based on scores for items including disposition, virtue, personality, cognitive faculty, and personal environments. The disposition refers to a property regarding consistency, the virtue refers to a property regarding morality, the personality refers to a property regarding empathy, the cognitive faculty refers to a property regarding problem-solving ability, and the personal environments refer to a property related to social support.

Furthermore, the processor may include an ethicality determination unit measuring scores for respective ethicality-related items of the artificial intelligence model based on the consultation content between the artificial intelligence model and the user. The ethicality-related items of the artificial intelligence model may include interpretability, transparency, responsibility, bias, and stability.

The ethicality determination unit may measure the ethicality score of the artificial intelligence model based on a user's survey regarding results output by the artificial intelligence model or measure the ethicality score of the artificial intelligence modelbased on analysis of the user's condition changes after the consultation with the artificial intelligence model.

In another aspect of the present disclosure, a control method of an electronic device according to various embodiments of the present disclosure may include: performing consultation between an artificial intelligence model and a user; identifying the user type based on the consultation content; andmeasuring an ethicality degree of result data output by the artificial intelligence model according to pre-stored evaluation criteria, wherein the ethicality degree is measured according to the identified user type.

In another aspect of the present disclosure, a computer program stored in a computer-readable recording medium according to an embodiment of the present disclosure may be executed by a processor of an electronic device to perform the above control method.

### Mode for Invention

The automatic evaluation execution unit 162 may analyze the response method output by the artificial intelligence model following the input of a problematic statement by the user, and perform an evaluation operation based on the analysis.
For example, the automatic evaluation unit 162 may analyze the response output by the artificial intelligence model in response to the input to evaluate whether an attempt was made to correct a problematic statement (e.g., illegal content, prohibited words such as profanity, or expressions indicating suicidal thoughts) entered by a user, and to what extent such correction was made, and then, based on the analysis, may assign a score regarding the accountability or stability of the artificial intelligence model. In this case, the automatic evaluation unit 162 may determine whether a consultation phrase opposite in meaning to the problematic statement input by the user was output, in order to determine whether a corrective attempt was made.

The type-based classification unit 163 may classify the evaluation information obtained by the user evaluation acquisition unit 161 and the automatic evaluation unit 162 according to the type of the user receiving the consultation. In this instance, the user type refers to the type identified by the user determination unit 140.

According to an embodiment of the present disclosure, since the user determination unit 140 classifies the users into three types, the evaluation information may be classified into three categories based on the user types.

The evaluation score calculation unit 164 may calculate an evaluation score for the artificial intelligence model for each user type based on the evaluation information classified into three categories by the type-based classification unit 163.

In this case, the evaluation operation may be performed not only on the artificial intelligence model, but also on each piece of output data (e.g., individual solution)or a specific unit of consultation content output by the artificial intelligence model. Accordingly, evaluation scores may also be calculated for each piece of output data or for each unit of consultation content.

The evaluation results of the artificial intelligence model may be used to improve the artificial intelligence model in the future. Furthermore, the evaluation results of the artificial intelligence model evaluated by user types may be used to generatea consultation model specialized for each user type.

According to various embodiments, the evaluation score calculation unit 164 may determine consultation topics and fields in which the evaluation score deviation by user types is greater than the reference value, and topics and fields in which the evaluation score deviation is less than the reference value. Moreover, in consultation topics and fields in which the evaluation score deviation by user types is greater than the reference value, the evaluation score calculation unit 164 may identify the result data (or the corresponding type) of the artificial intelligence model that received high evaluation (e.g., exceeding the reference score) for each user type, and support the consultation performing unit 150 to apply the identified result data in future consultations.

Accordingly, when a consultation is conducted in consultation topics and fields in which the evaluation score deviation by user types is greater than the reference value, the consultation performing unit 150 may apply the artificial intelligence model (or corresponding result data type of the artificial intelligence model) that received high evaluation for the identified user type after previously identifying the user type. As a result, even if a certain response from the artificial intelligence model may induce negative changes or be perceived as untrustworthy for other user types, if the response is evaluated to induce positive changes or be trustworthy for the identified user type, the consultation performing unit 150 may control the corresponding response to be output in the consultation process for the corresponding user.

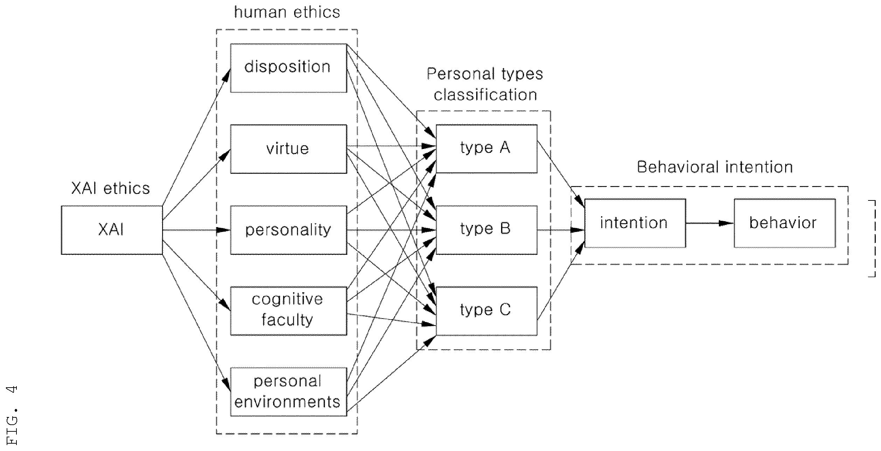

FIG. 4 is a diagram for depicting an evaluation operation for output data of an artificial intelligence model according to user types according to an embodiment of the present disclosure.

As illustrated in FIG. 4, the artificial intelligence (XAI) model that performs consultation or the result data output by the artificial intelligence model according to an embodiment of the present disclosure may be evaluated for the ethicality or explainability of intention based on three user types classified according to scores in five evaluation items for the user.

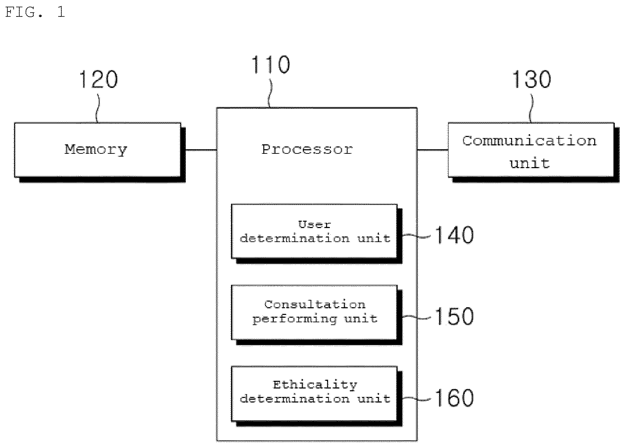

As a reference, the memory 120, which is one of the components of the electronic device 100 of FIG. 1, may store commands and algorithms required to perform overall operations according to an embodiment of the present disclosure. The memory 120 may store artificial intelligence models necessary for identifying the user type and conducting consultation with the user based on the identified type.

The communication unit 130 may support communication operations with various user terminals for acquiring user input. For instance, the communication unit 130 may receive voice data or text data for consultation with the user via a separate user terminal. Furthermore, the communication unit 130 may receive evaluation data from the user terminal regarding the artificial intelligence model or the result data output by the artificial intelligence model.

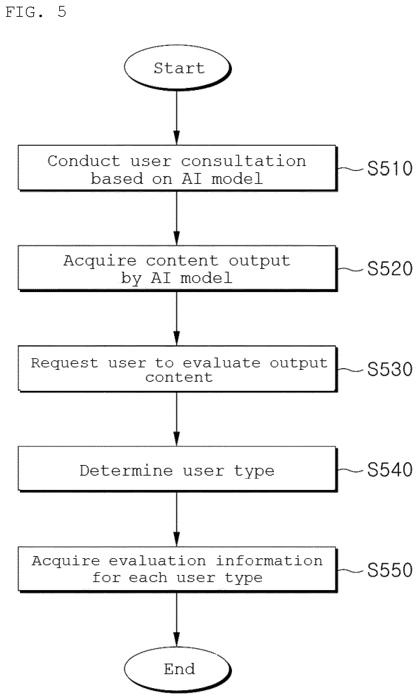

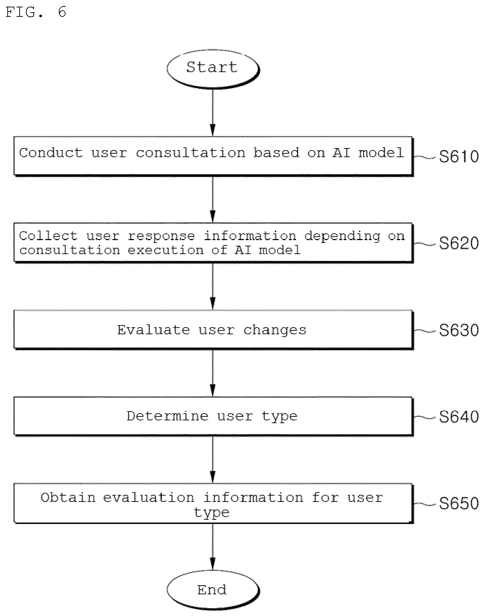

FIGS. 5 and 6 are diagrams illustrating the evaluation operation of the artificial intelligence model of the electronic device according to an embodiment of the present disclosure.

FIG. 5 illustrates a method of evaluating the artificial intelligence model based on content directly assessed by the user after confirming the output result of the model, and FIG. 6 illustrates a method of evaluating the artificial intelligence model based on user changes identified through user responses during the consultation process.

Referring to each drawing, the methods will be described in detail.

As illustrated in FIG. 5, the electronic device 100 according to an embodiment of the present disclosure may perform operation S510 for conducting a user consultation based on the artificial intelligence model.

Subsequently, the electronic device 100 may perform operation S520 for acquiring content output by the artificial intelligence model during the consultation.

Then, the electronic device 100 may perform operation S530 of requesting the user to evaluate the content output by the artificial intelligence model.

Thereafter, the electronic device 100 may perform operation S540 of determining the user type according to preset evaluation criteria.

Subsequently, the electronic device 100 may perform operation S550 of acquiring evaluation information for each user type. In this instance, the operation S540 of determining the user type may be performed at any point from the beginning of the consultation using the artificial intelligence model to a point before the generation of evaluation information for each user type.

FIG. 6 illustrates a sequence of operations in which the evaluation of output results from an artificial intelligence model is automatically performed based on the consultation content, without relying on a user's evaluation.

As illustrated in FIG. 6, after performing operation S610 in which the AI model-based consultation is conducted, the electronic device 100 according to an embodiment of the present disclosure may proceed to operation S620 to collect consultation content and user response information generated during the consultation process.

Subsequently, the electronic device 100 may perform operation S630 to evaluate user changes based on the collected consultation content. In this instance, the user changes may include, for example, an increase in positivity or negativity in a specific field of the user.

In addition, the electronic device 100 may perform operation S640 to determine the user type through the consultation content. As described with reference to FIG. 5, the timing of the operation for determining the user type may vary.

Afterward, the electronic device 100 may perform operation S650 to obtain evaluation information for each user type.

In summary, the electronic device according to an embodiment of the present disclosure may include:a memory that stores evaluation criteria related to the ethicality of an artificial intelligence model performing consultation; anda processor that measures an ethicality degree of result data output by the artificial intelligence model according to the evaluation criteria, wherein the ethicality degree is measured according to user types of users consulting with the artificial intelligence model.

Moreover, the processor may include a user determination unit that measures scores for respective ethicality-related items of the user based on the consultation content between the artificial intelligence model and the user, and classifies the user byuser types based on the item scores.

In this instance, the user types may be classified based on scores for items including disposition, virtue, personality, cognitive faculty, and personal environments. The disposition refers to a property regarding consistency, the virtue refers to a property regarding morality, the personality refers to a property regarding empathy, the cognitive faculty refers to a property regarding problem-solving ability, and the personal environments refer to a property related to social support.

Furthermore, the processor may include an ethicality determination unit measuring scores for respective ethicality-related items of the artificial intelligence model based on the consultation content between the artificial intelligence model and the user. The ethicality-related items of the artificial intelligence model may include interpretability, transparency, responsibility, bias, and stability.

Additionally, the ethicality determination unit may measure the ethicality score of the artificial intelligence model based on a user's survey regarding results output by the artificial intelligence model or measure the ethicality score of the artificial intelligence modelbased on analysis of the user's condition changes after the consultation with the artificial intelligence model.

In another aspect of the present disclosure, a control method of an electronic device according to various embodiments of the present disclosure may include: performing consultation between an artificial intelligence model and auser; identifying the user type based on the consultation content; andmeasuring an ethicality degree of result data output by the artificial intelligence model according to pre-stored evaluation criteria, wherein the ethicality degree is measured according to the identified user type.

In another aspect of the present disclosure, a computer program stored in a computer-readable recording medium according to an embodiment of the present disclosure may be executed by a processor of an electronic device to perform the above control method.

The ethicality evaluation operation performed by the electronic device 100 according to various embodiments of the present disclosure may be applied not only in psychological consultation but also in various fields such as efficient learning, advanced AI-based decision-making (cognition, judgment, reasoning), trustworthy and safe AI, and industrial applications of AI.

Moreover, according to various embodiments, each item of theexplainable artificial intelligence (XAI) in the artificial intelligence model for ethicality evaluation may be designed to influence the ethical characteristics of the user.

Furthermore, according to various embodiments, the electronic device 100 may support users in evaluating the ethicality scores of the artificial intelligence model.

Additionally, according to various embodiments, the electronic device 100 may determine the importance of ethicality items in the artificial intelligence model to vary depending on the user type. In other words, in the ethicality evaluation method of the artificial intelligence model, the ethicality items that are considered important may differ depending on the characteristics of the user.

According to various embodiments, during the consultation operation, the electronic device 100 may conduct consultation not only based on the user's psychological characteristics but also based on a multidimensional evaluation including morality, problem-solving ability, and social behavior.

According to various embodiments, the electronic device 100 may identify artificial intelligence models with relatively high ethicality evaluation scores (i.e., high explainability). The artificial intelligence models with high scores may promote more active user engagement than black-box type models and may also enhance the effectiveness of the consultation.

According to an embodiment of the present disclosure, the electronic device 100 may include a processor 110, a memory 120, and a communication unit 130.

The memory 120 may store various programs and data necessary for the operation of the electronic device. The memory 120 may be implemented as non-volatile memory, volatile memory, flash memory, a hard disk drive (HDD), or a solid state drive (SSD).

The communication unit 130 may perform communication with an external device. In particular, the communication unit 130 may include various communication chips such as a Wi-Fi chip, a Bluetooth chip, a wireless communication chip, an NFC chip, and a low-power Bluetooth chip (BLE chip). At this time, the Wi-Fi chip, Bluetooth chip, and NFC chip communicate in the LAN mode, Wi-Fi mode, Bluetooth mode, and NFC mode, respectively. When using the Wi-Fi chip or Bluetooth chip, various pieces of connection information such as SSID and session key are first transmitted and received. After establishing a communication link using this information, various pieces of information may be sent and received. The wireless communication chip refers to a chip that performs communication according to various communication standards such as IEEE, Zigbee, 3G (3rd Generation), 3GPP (3rd Generation Partnership Project), and LTE (Long Term Evolution).

The processor 110 may control the overall operation of the user device using various programs stored in the memory 120. The processor may include RAM, ROM, a graphics processing unit, a main CPU, first to nth interfaces, and buses. At this time, the RAM, ROM, graphics processing unit, main CPU, first to nth interfaces, etc. may be connected to each other via a bus.

The RAM stores O/S and application programs. Specifically, when the electronic device boots up, the O/S may be stored in the RAM, and various application data selected by the user may be stored in the RAM.

The ROM stores a command set for system booting, etc. When a turn-on command is input and power is supplied, the main CPU copies the O/S stored in the memory 200 to the RAM according to the command stored in the ROM and executes the O/S to boot the system. When booting is complete, the main CPU copies various application programs stored in the memory 200 to the RAM and executes the application programs copied to the RAM to perform various operations.

The main CPU accesses the memory 120 and performs booting using the OS stored in the memory 120. Further, the main CPU performs various operations using various programs, contents, data, etc. stored in the memory 120.

The first to n interfaces are connected to the various components described above. One of the first to n interfaces may be a network interface connected to the external device via a network.

Meanwhile, the processor may control the artificial intelligence model. In this case, it goes without saying that a control unit may include a graphics-dedicated processor (e.g., GPU) for controlling the artificial intelligence model.

The processor 110 may include one or more cores (not shown) and a graphics processing unit (not shown) and/or a connection path (e.g., a bus, etc.) for transmitting and receiving signals with other components.

The processor according to an embodiment performs a method described in connection with the present disclosure by executing one or more instructions stored in the memory.

Meanwhile, the processor 110 may further include RAM (Random Access Memory, not shown) and ROM (Read-Only Memory, not shown) for temporarily and/or permanently storing a signal (or data) processed within the processor. Further, the processor may be implemented in the form of a system on chip (SoC), which includes at least one of a graphics processing unit, RAM, and ROM.

The memory 120 may store programs (one or more instructions) for processing and controlling the processor 110. Programs stored in the storage may be divided into multiple modules according to their functions.

The steps of the method or algorithm described in connection with an embodiment of the present disclosure may be implemented directly in hardware, in a software module executed by hardware, or through a combination thereof. The software module may reside in a random access memory (RAM), a read only memory (ROM), an erasable programmable ROM (EPROM), an electrically erasable programmable ROM (EEPROM), a flash memory, a hard disk, a removable disk, a CD-ROM, or any other form of computer readable recording medium that is well known in the art to which the present disclosure pertains.

The components of the present disclosure can be implemented as a program (or application) to be executed in combination with a computer, which is hardware, and stored in a medium. The components of the present disclosure may be implemented by software programming or software elements. Similarly, the embodiment may be implemented in a programming or scripting language, such as C, C++, Java, or assembler, including various algorithms implemented as a combination of data structures, processes, routines, or other programming constructs. The functional aspects may be implemented as algorithms executed on one or more processors 100.

Although the present disclosure has been described above with reference to preferred embodiments, it will understood by those skilled in the art that various modifications and changes may be made to the present disclosure without departing from the scope of the present disclosure. It should be noted that in order to achieve the intended effect of the present disclosure, it is not necessary to separately include all the functional blocks illustrated in the drawings or follow all the sequences illustrated in the drawings in the exact order depicted, and even if not, it may fall within the technical scope of the present disclosure described in the claims.

## Claims

1. An electronic device comprising:
a memory that stores evaluation criteria related to the ethicality of an artificial intelligence model performing consultation; and
a processor that measures an ethicality degree of result data output by the artificial intelligence model according to the evaluation criteria, wherein the ethicality degree is measured according to user types of users consulting with the artificial intelligence model.

2. The electronic device according to claim 1, wherein the processor includes a user determination unit that measures scores for respective ethicality-related items of the user based on the consultation content between the artificial intelligence model and the user, and classifies the user into a user type based on the item scores.

3. The electronic device according to claim 2, wherein the user typesare classified based on scores for items including disposition, virtue, personality, cognitive faculty, and personal environments.

4. The electronic device according to claim 1, wherein the processor includes an ethicality determination unit measuring scores for respective ethicality-related items of the artificial intelligence model based on the consultation content between the artificial intelligence model and the user, and
whereinthe ethicality-related items of the artificial intelligence model may include interpretability, transparency, responsibility, bias, and stability.

5. The electronic device according to claim 4, wherein the ethicality determination unit measures the ethicality score of the artificial intelligence model based on a user's survey regarding results output by the artificial intelligence model.

6. The electronic device according to claim 4, wherein the ethicality determination unit measures the ethicality score of the artificial intelligence modelbased on analysis of the user's condition changes after the consultation with the artificial intelligence model.

7. A control method of an electronic device comprising the steps of:
performing consultation between an artificial intelligence model and a user;
identifying the user type based on the consultation content; and
measuring an ethicality degree of result data output by the artificial intelligence model according to pre-stored evaluation criteria, wherein the ethicality degree is measured according to the identified user type.

8. Acomputer program stored in a computer-readable recording medium which is executed by a processor of an electronic device to perform the control method according to claim 7.
